# EUROPÄISCHE PATENTSCHRIFT

(11) **EP 1 854 621 B1**
(45) Veröffentlichungstag und Bekanntmachung des Hinweises auf die Patenterteilung: **03.08.2011**
(21) Anmeldenummer: 07009276.2
(22) Anmeldetag: 09.05.2007
(51) Int. Cl.: B29C 70/30, B29C 70/86, A61F 2/50, A61F 2/78, A61F 2/80, B29C 70/54

(54) **Verfahren zum Giessen eines Prothesenschaftes**
Method for casting a prosthesis shaft
Procédé de moulage d'une emboîture

(30) Priorität: 09.05.2006 DE 102006021857
(43) Veröffentlichungstag der Anmeldung: 14.11.2007
(73) Patentinhaber: Otto Bock HealthCare IP GmbH & Co. KG, 37115 Duderstadt (DE)
(72) Erfinder: Becker, Karl, 37115 Duderstadt (DE); Anhalt, Klaus-Peter, 37434 Rhumspringe (DE)
(74) Vertreter: Lins, Martina

(56) Entgegenhaltungen:
- WO-A2-03/034904
- AT-B- 369 978
- US-A- 4 635 626
- US-A- 5 156 631
- US-A1- 2005 149 202

## Beschreibung

Die Erfindung betrifft ein Verfahren zum Gießen einer Halterung, die am menschlichen Körper anzubringen ist, in Form eines Prothesenschaftes oder eines Orthesenteils mit innen liegender, gitter- oder rahmenförmiger Verstärkung.

Unter einer Prothese verstehen wir den künstlichen Ersatz für eine amputierte Gliedmaße, die an der Restgliedmaße und zwar insbesondere dem Amputations-Stumpf festgemacht wird. Hierfür besitzt die Prothese eine trichter- oder tassenförmige Aufnahme für den Stumpf, im Folgenden als Prothesenschaft bezeichnet. Der Schaft soll passgenau am Stumpf anliegen und eine optimale Kraftübertragung vom Körper des Amputierten auf die Prothese ermöglichen ohne Druck- oder Scheuerstellen zu erzeugen. An Maßänderungen geringeren Ausmaßes (Schwellen, Abschwellen des Stumpfes) sollte der Schaft sich nach Möglichkeit elastisch anpassen können.

Ähnliche Anforderungen sind an sogenannte Orthesen bzw. deren körpernahe, halternde Teile zu stellen. Unter Orthesen werden den Körper oder Gliedmaßen des Körpers von außen stützende Halteapparate verstanden, häufig auch als Stützapparate oder Schienen bezeichnet.

Um den Anforderungen gerecht zu werden, werden Schäfte wie andere orthopädische Halterungen (Orthesen) mit direktem Körperkontakt im Allgemeinen aus festen, formstabilen, aber zumindest bereichsweise dennoch elastischen oder weich nachgiebigen Kunststoffen gefertigt.

Hierfür ist es auch üblich, verschiedene Kunststoffe in mehreren Schichten zu kombinieren und innerhalb der Kunststoffe Verstärkungen, z.B. aus textilen Geweben oder Gewirken oder aus Carbonfasern (i. a. in Form von Carbonfasermatten) vorzusehen. Auch können die Verstärkungen gezielt in bestimmten Zonen des Halterungsbauteils eingesetzt werden, oder es können bestimmte, z.B. besonders weiche Materialien in ausgewählten Bereichen anstelle des Grundmaterials oder zusätzlich, pflasterartig eingebracht werden (Polster, Patches).

Zum Aufbau von Laminaten mit polymergetränkten textilen Verstärkungen sind grundsätzlich Wickeltechniken oder Gusstechniken üblich. Diese Verfahren sind dem Orthopädietechniker aus der Praxis bekannt.

Bei der Wickeltechnik werden mit einem noch nicht ausreagierten Polymer getränkte Bandagen auf eine Form oder gegebenenfalls auf den Stumpf bzw. die Gliedmaße direkt gewickelt und die gewickelte Hülle wird aushärten gelassen. Alternativ können auch getränkte textile Strümpfe in einer oder in mehreren Schichten auf eine Form aufgezogen werden. Diese Technik wird häufig auch für Gipsaufbauten einschließlich Gips-Modellformen (Positivabguss, Negativabguss) verwendet.

Bei der Gusstechnik wird ein noch nicht ausgehärtetes bzw. ausreagiertes, flüssiges Polymer in eine geeignete Schaft- oder Halterungs-Hohlform eingegossen, die aus der Positivform des Stumpfes und einer mit Hilfe eines Abstandsmediums über der Positivform erhaltenen Negativform gebildet werden kann. Das Abstandsmedium wird entweder vor dem Guss entfernt (ungefüllter Formguss) oder kann im Falle eines porösen Abstandsmediums mit eingebunden, d.h. eingegossen werden, so dass ein gefülltes oder verstärktes Bauteil entsteht. Insbesondere kann das Abstandsmedium ein poröser Füllstoff (ausgehärteter Schaum, verfestigtes Granulat) sein oder aus textilen oder anderen Flüssigkeit aufnehmenden Schichten aufgebaut sein (gewickelte Bandagen, Strümpfe, Socken, allgemein Textillagen). Um das Gießen in enge Hohlformen zu erleichtern, wird häufig eine Saugtechnik angewendet: Während die flüssige und im Allgemeinen viskose Polymervorstufe (Eduktmischung, Präpolymere) in die Hohlform eingegossen oder -gespritzt wird, wird aus demselben Volumen (im Allgemeinen an anderer Stelle über ein Ventil) Luft abgesaugt, um das Einfließen der Flüssigkeit zu erleichtern.

Sehr zähe Materialien, die nicht gegossen werden können, werden häufig auf eine Positivform oder darüber liegende Textil- bzw. Verstärkungslagen aufgestrichen.

Aus der EP 201 884 A1 ist ein Verfahren zur Herstellung einer Prothese mit einem Schaft bekannt, bei dem Polster aus einem viskoelastischen Material in eine konventionelle Wickel-Guß-Technik einbezogen werden. Der Schaft aus den getränkten Wickelbandagen ist relativ steif. Die Polster können Druckstellen mindern, jedoch Druck- und Scherkräfte wegen mangelnder Elastizität nicht optimal weiterleiten.

Aus der EP 650 708 B1 ist ein vielstufiges Verfahren zum Herstellen eines Schafts und einer Einlage bekannt, das u. a. einen Vollkontakt zwischen Schaft und Stumpf sowie eine bessere Stoß- und Scherkraftübertragung sichern soll. Die weiche elastische Einlage besteht aus einem viskoelastischen Polyurethan, das aus einem aromatischen Diisocyanat und-elastisch machenden Polyolen hergestellt wurde. Die zweiteilige Form aus Schaft und indviduell auf dem Stumpf dehnbar geformter Einlage ist für den Benutzer mühsam anzulegen und wird oft als einengend empfunden. Die Einlage erübrigt nicht, dass auch der Schaft für höheren Komfort mit unterschiedlich steifen, elastischen und weichen Bezirken komplexer gestaltet werden sollte.

Der Anmelderin sind aus ihrer Praxis Schäfte aus formstabilem, jedoch unter Druck reversibel verformbarem weich-elastischen Polyurethan mit innen liegender Verstärkung in Form eines steiferen Rahmens oder Gitters bekannt. Den Rahmen bildet beispielsweise eine carbonfaser-verstärkte gefensterte Polyacrylatschicht. Bei derartigen aus mehreren Einzelschichten gefertigten Schäften hat sich als nachteilig herausgestellt, dass die Schichten sich insbesondere an den Rändern voneinander lösen können und der Schaft mit der Zeit unbrauchbar wird.

Der Erfindung liegt daher die Aufgabe zugrunde, einen Schaft aus bereichsweise unterschiedlichen Materialien, bei dem eine Verstärkung von einem Polyurethan umschlossen ist, so auszugestalten und ein für dessen Herstellung geeignetes Verfahren bereitzustellen, dass Ablösungen zwischen einzelnen Schichten vermieden werden.

Zur Lösung dieser Aufgabe ist ein Verfahren der eingangs genannten Art, bei welchem über der Form eines Gliedmaßenteils oder -stumpfes nacheinander eine erste Polyurethan-Lage, die ein im Guss verarbeitbares Polyurethan (PU) enthält, darauf die Verstärkung und darauf eine weitere Polyurethan-Lage aufgebracht werden, durch die besondere Verfahrensweise der nachfolgend beschriebenen schritte a) bis g) gekennzeichnet.

Das Verfahren zum Gießen einer am menschlichen Körper anzubringenden Halterung in Form eines Prothesenschaftes oder eines Orthesenteils mit in Ihrem Kernbereich liegender, gitter- oder rahmenförmiger Verstärkung und dem grundsätzlichen Schichtaufbau: unterliegende Polyurethan(PU)-Lage, Verstärkung im Kernbereich, aufliegende Polyurethan(PU)-Lage, sieht demnach vor, dass mit den folgenden Schritten in der angegebenen Reihenfolge:
a) an der Position einer der Verstärkung unterliegenden PU-Lage ein mit dem noch nicht ausreagierten Polyurethan tränkbares Platzhaltermaterial in Form der gewünschten gliedmaßenseitigen PU-Lage aufgebracht wird,
b) darauf, d.h. um das Platzhaltermaterial herum oder auch als Schale über dieser Lage, die gitter- oder rahmenförmige Verstärkung erstellt wird,
c) die Verstärkung abgenommen wird,
d) eine erste Polyurethanlage unter Durchtränken des Platzhaltermaterials gegossen wird,
e) die Verstärkung auf die Polyurethanoberfläche der ersten Polyurethanlage in einem Polymerisationszustand nach Erreichen der Formstabilität wieder aufgesetzt wird,
f) ein weiteres mit dem noch nicht ausreagierten Polyurethan tränkbares Platzhaltermaterial in Form der gewünschten weiteren, die Verstärkung umgebenden PU-Lage aufgebracht wird und
g) die weitere PU-Lage unter Durchtränken des zweiten Platzhaltermaterials gegossen wird, so dass sich die beiden PU-Lagen dort wo sie in Kontakt treten beim Aushärten des Polyurethans der aufliegenden PU-Lage mit Hilfe eines zwischen den Lagen aufgebrachten Primers und/oder durch gemeinsames Aushärten beider PU-Lagen reaktiv verbinden und beide PU-Lagen die Verstärkung einschließen.

Wie üblich wird das PU-Gießharz als Mehrkomponentensystem bereitgestellt, wobei die Komponenten unmittelbar vor Anwendung, d.h. Gießen bzw. Tränken des Platzhaltermaterials, vermischt werden. Eine dieser Komponenten enthält vorzugsweise ein Präpolymer wenigstens eines Isocyanates, wobei aliphatische Isocyanate oder vorwiegend aliphatische Isocyanate bevorzugt sind.

Die Rohstoffe für die Polyurethan-Masse werden so gewählt, dass die Masse gut gießfähig ist und ein Abbindeverhalten zeigt, bei dem die Masse nach Erreichen der Formstabilität für wenigstens ein bis zwei Stunden nicht vollständig durchpolymerisiert ist. Die Auswahl erfolgt durch Vorversuche im Rahmen der oben grundsätzlich angegebenen Zusammensetzung. Die Masse kann eingefärbt sein, z.B. hautfarben, wie grundsätzlich im Stand der Technik bekannt.

Je nach Abbindeverhalten des Gießharzes, Komplexität des Halterungsaufbaus und anderen anwendungsspezifischen Vorgaben können verschiedene Varianten der Erfindung jeweils besonders vorteilhaft sein.

Gemäß einem Aspekt der Erfindung werden die Schritte a) bis d) wie oben beschrieben durchgeführt und die Schritte e) bis g) in folgender Weise:
e) die Verstärkung wird auf die freie Polyurethanoberfläche der ersten Polyurethanlage in einem Polymerisationszustand nach Erreichen der Formstabilität und vor dem Ausreagieren wieder aufgesetzt,
f) ein weiteres mit dem noch nicht ausreagierten Polyurethan tränkbares Platzhaltermaterial in Form der gewünschten weiteren PU-Lage wird aufgebracht und
g) die weitere PU-Lage wird unter Durchtränken des zweiten Platzhaltermaterials gegossen, so dass sich die beiden PU-Lagen dort wo sie in Kontakt treten beim Aushärten des Polyurethans beider PU-Lagen reaktiv verbinden und die Verstärkung einschließen.

Die oben beschriebene Variante ist besonders vorteilhaft bei langsam aushärtendem Gießharz oder wenn die technischen Möglichkeiten ein besonders schnelles Arbeiten ermöglichen.

Gemäß einem weiteren Aspekt der Erfindung werden die Schritte a) bis d) wiederum wie oben angegeben ausgeführt und die Schritte e) bis g) wie nachfolgend angegeben:
e) die Verstärkung wird auf die Polyurethanoberfläche der ersten Polyurethanlage in einem Polymerisationszustand nach Erreichen der Formstabilität wieder aufgesetzt und wenigstens auf dem Randbereich der ersten PU-Lage und außerhalb der Fläche, auf der die Verstärkung aufliegt, wird ein Primer aufgetragen, ,
f) ein weiteres mit dem noch nicht ausreagierten Polyurethan tränkbares Platzhaltermaterial in Form der gewünschten weiteren PU-Lage wird aufgebracht und
g) die weitere PU-Lage wird unter Durchtränken des zweiten Platzhaltermaterials gegossen, so dass sich die beiden PU-Lagen dort wo sie in Kontakt treten beim Aushärten des Polyurethans der aufliegenden PU-Lage mit Hilfe des zwischen den PU-Lagen aufgebrachten Primers reaktiv verbinden und die Verstärkung einschließen.

Die Verwendung des Primers gemäß dieser Variante bietet sich besonders an, wenn das Gießharz schnell ausreagiert oder die Bearbeitung der Verstärkung längere Zeit in Anspruch nimmt, so dass die erste oder unterliegende PU-Lage schon ausgehärtet oder weitgehend ausgehärtet ist. Die Anbindung der zweiten PU-Lage erfolgt dann mit Hilfe des Primers während des Ausreagierens der zweiten oder aufliegenden PU-Lage.

Das Polyurethan für die die Verstärkung umgebenden Lagen kann aromatische oder aliphatische Isocyanate enthalten, und ist vorzugsweise ein solches auf Basis aliphatischer Isocyanate, weiter vorzugsweise HDI, was bedeutet, dass wenigstens 50 Gew.-%, weiter vorzugsweise wenigstens 70 Gew.-% der Isocyanate des PU-Gießharzes aliphatischer Natur sein sollten.

Der Aufbau des Schaftes oder der Halterung über der Stumpf- bzw. Gliedmaßenform erfolgt erfindungsgemäß in mehreren Schritten und wird im Allgemeinen auf einem zuvor angefertigten Positivmodell (z.B. Gipsmodell) des Gliedmaßenteils oder Stumpfes durchgeführt.

Die die Verstärkung umgebenden PU-Lagen können, falls dies gewünscht wird, ihrerseits von weiteren Lagen umgeben sein. Hierfür kommen verschiedene Coatings, aber auch nach außen gerichtete, zusätzliche schützende Lagen, wie z.B. textile Abdeckungen und dergleichen in Frage. Sofern im Innern des Schafts weitere Lagen vorgesehen wären, würden diese zunächst auf die Positivform aufgebracht. Ansonsten beginnt man mit Schritt a), nachdem gegebenenfalls ein Trennmittel oder eine Trennfolie auf die Positivform aufgebracht wurde.

Als Schritt a) wird an der räumlichen Position, an der die der Verstärkung unterliegenden PU-Lage vorgesehen ist, ein mit dem noch nicht ausreagierten Polyurethan (d.h. der vorbereiteten, gießfähigen PU-Masse, die auch als Gießharz bezeichnet wird) tränkbares Platzhaltermaterial in Form der gewünschten gliedmaßenseitigen PU-Lage aufgebracht. Durch dieses Platzhaltermaterial wird die Form der späteren PU-Lage modelliert bzw. vorgegeben. Je nach Form der späteren Halterung und des Gliedmaßenbereichs nimmt das Platzhaltermaterial die Form einer Lage (Schicht), Manschette oder Tasse ein.

In Weiterbildung der Erfindung kann für den Gießvorgang um das Platzhaltermaterial herum eine Trennfolie gelegt werden, die nach dem Aushärten des Gießharzes oder nach Erreichen der Formstabilität der Lage abgezogen wird. Eine solche Trennfolie wäre nicht nötig, wenn das Platzhaltermaterial alternativ durch Begießen von außen durchtränkt werden könnte ohne dass die Gießmasse abtropft. Bei den derzeit bevorzugten Ausführungsbeispielen wird hingegen jeweils eine Trennfolie verwendet, und das Hineinfließen der Gussmasse in den Zwischenraum zwischen Positivform oder unterliegenden Schichten einerseits und Folie andererseits wird gegebenenfalls durch Absaugen der verdrängten Luft unterstützt. Solche Gießverfahren sind dem Fachmann grundsätzlich bekannt.

In Schritt b) wird danach um das Platzhaltermaterial herum, d.h. über der späteren ersten PU-Lage, die gitter- oder rahmenförmige Verstärkung erstellt.

Diese gitter- oder rahmenförmige Verstärkung ist vorzugsweise aus einem Hartpolymer, d.h. allgemein einem Werkstoff mit einer höheren Steifigkeit als das vorgesehene Polyurethan. Besonders geeignet sind faserverstärkte Kunststoffe. Gemäß einer bevorzugten Ausführungsform der Erfindung ist die gitter- oder rahmenförmige Verstärkung eine nach dem Aushärten gefensterte über der ersten PU-Lage geformte oder gegossene verstärkte Polyacrylatlage sein. Die Herstellung derartiger Polyacrylat-Verstärkungen ist dem Fachmann bekannt. Die Lage wird zunächst in Form einer harten Schale über die darunter liegende PU-Schicht laminiert oder auch gegossen, nach dem Aushärten abgenommen und an den Stellen, die innerhalb des Schaftes weniger steif sein sollen gefenstert, d.h. ausgeschnitten. Die gitterartige Verstärkung könnte jedoch auch anders hergestellt werden. Beispielsweise könnten über einer Trennfolie Stege aus einem geeigneten im ausgehärteten Zustand genügend steifen Material auf der unterliegenden PU-Lage aufgespritzt werden, metallische Schienen könnten ebenfalls einbezogen werden, z.B. auf eine gitterförmige textile Schicht aufgeklebt werden, usw. Vorzugsweise ist die gefensterte Polyacrylatlage carbonfaserverstärkt. Hierfür wird das Acrylharz auf Carbonfasermatten bzw. -bandagen aufgegossen.

Nachdem nun die Verstärkung in Schritt c) abgenommen wurde, kann in Schritt d) eine erste Polyurethanlage gegossen werden, und zwar entweder in bevorzugter Ausführung unter Durchtränken des Platzhaltermaterials oder indem um das Platzhaltermaterial herum in üblicher Weise eine Negativform erstellt wird, das Platzhaltermaterial danach entfernt wird und in den Zwischenraum zwischen Negativform und Unterlage der Schicht eine Polyurethan-Gussmasse für eine reine, unverstärkte Lage eingegossen wird. Ein entsprechendes Verfahren ist beispielsweise in der EP 650 708 für den Guss einer Schafteinlage beschrieben.

Das Platzhaltermaterial ist vorzugsweise ein in wenigstens einer Schicht aufgebrachtes textiles, weiter vorzugsweise gewirktes Material. Dieses Material wird bevorzugt mit der Gussmasse gefüllt, so dass ein Laminat entsteht.

Insbesondere kann das textile Material strumpf- oder sockenförmig sein, wobei vorzugsweise mehrere Strümpfe oder Socken übereinander gezogen werden. Ein elastisch gewirktes Material ist dabei besonders vorteilhaft. Es können auch Binden verwendet werden.

Alternativ kann das Platzhaltermaterial aus einer porösen Manschette bestehen, vorzugsweise aus einem Polyurethanschaum. Auch ein solches offenzelliges poröses Material kann mit dem PU-Gießharz gefüllt werden. Andere Platzhaltermaterialien sind denkbar, sofern sie den hier beschriebenen Zweck erfüllen.

Noch vor dem Ausreagieren des PU-Gießharzes wird nun die in der Regel vorhandene Trennfolie oder Gussbegrenzungslage abgezogen, sobald dies möglich ist und die PU-Lage dabei unverändert und stabil bleibt. Anschließend wird in Schritt e) die zuvor abgenommene Verstärkung auf die Polyurethanoberfläche der ersten Polyurethanlage wieder aufgesetzt. Die Polyurethanlage befindet sich dabei in einem Polymerisationszustand nach Erreichen der Formstabilität.

Sofort wird nun in Schritt f) ein weiteres mit dem noch nicht ausreagierten Polyurethan tränkbares Platzhaltermaterial für eine weitere die Verstärkung umgebenden PU-Lage aufgebracht wie zuvor für die erste PU-Lage, und anschließend wird in Schritt g) die weitere PU-Lage in entsprechender Weise wie für die erste Lage beschrieben gegossen, d.h. vorzugsweise unter Durchtränken des zweiten Platzhaltermaterials, so dass sich die beiden PU-Lagen dort wo sie in Kontakt treten beim Aushärten des Polyurethans beider PU-Lagen oder alternativ beim Aushärten der aufliegenden PU-Lage, die angrenzt an die schon gegossene, im PU-PU-Kontaktbereich mit Primer behandelte Lage reaktiv verbinden.

Die zweite Platzhaltermaterial-Lage wird in der gleichen Weise aufmodelliert wie zuvor die erste Lage und auch das Gießen erfolgt in der oben für die erste Lage schon beschriebenen Weise.

Das Verfahren bewirkt, dass sich die beiden Polyurethan-Lagen, die die Verstärkung einschließen, wie eine zusammenhängende Lage verhalten, da das Polyurethan vorzugsweise gemeinsam durchhärtet. Im zweiten Fall, d.h. bei Verwendung des Primers, findet während der Polymerisationsreaktion in wenigstens der aufliegenden PU-Lage zugleich eine durch den Primer bewerkstelligte zusätzliche Vernetzung zwischen den PU-Schichten statt. Im ersteren Fall durchdringt also die Polymerisationsreaktion nicht nur jeweils eine Schicht, sondern findet übergreifend in beiden Schichten und auch quer durch die Kontaktfläche statt. Die Verstärkung wird vollständig eingeschlossen und in den gefensterten Bereichen bzw. innerhalb der Maschen einer gitterartigen Verstärkung oder in einem Rahmeninneren von einer einheitlich durchpolymerisierenden Masse durchdrungen, und zwar entweder durch gemeinsames Aushärten der beiden noch nicht bzw. noch nicht voll ausgehärteten PU-Lagen oder alternativ oder zusätzlich durch das Aushärten der aufliegenden PU-Lage auf der zuvor gegossenen und mit Primer versehenen unteren PU-Lage. Es kann daher nicht zur Trennung der dem Schaftaufbau nach ursprünglich einzeln zu betrachtenden Schichten kommen.

Die Fläche, in der die Polyurethanschichten in Kontakt treten (Kontaktfläche), befindet sich im Allgemeinen am Schaftrand, d.h. dass die Verstärkung gegenüber den PU-Lagen verkürzt ist und sich nicht bis zum Schaftrand erstreckt, und auch innerhalb aller Ausnehmungen der Verstärkung, wie oben beschrieben.

Auf die Schaftaußenseite können weitere Schichten aufgebracht werden, z.B. eine dekorative Schicht, eine textile Abdeckung oder dergleichen.

Gemäß einer vorteilhaften Weiterbildung der Erfindung kann zusätzlich auf die gegitterte Verstärkung (d.h. im Allgemeinen rundum auf die fertiggestellte, abgenommene Verstärkung) ein Primer, Haftvermittler oder Klebstoff, vorzugsweise aus einer Polymerlösung, aufgebracht werden.

Derartige Primer sind dem Fachmann grundsätzlich für die Verbindung zwischen verschiedenen Kunststoffen bekannt. Häufig handelt es sich hierbei um ggf. funktionalisierte Polymere in organischen Lösungsmitteln, die den zu behandelnden Kunststoff aufquellen oder anlösen können. Es kann sich auch um Präpolymere in Substanz handeln, auch hier sind Mehrkomponentensysteme, die erst kurz vor Verwendung des Primers, Haftvermittlers oder Klebstoffs gemischt werden, möglich.

Sofern die Verstärkung aus oder mit Hilfe eines Acrylharzes erstellt wird, ist es besonders vorteilhaft in Kontakt mit der Acrylharz-Verstärkung einen Primer aus einem Lösungsmittel für thermoplastische Polyacrylate mit oder ohne darin gelöstem Polymer zu verwenden. Als Lösungsmittel für den Primer bieten sich alle organischen Lösungsmittel, die unter anderem PMMA lösen, an, bevorzugt organische Lacklösungsmittel, besonders bevorzugt 2-Butanon (Methylethylketon). Die Acrylharz-Verstärkung wird durch das Lösungsmittel Butanon angelöst und ermöglicht dadurch über einen Kaltverschweißungsvorgang die Haftung der Verstärkung mit der angrenzenden Lage vermittelt durch den Primer.

Neben dem Lösungsmittel, bei dem es sich auch um ein Lösungsmittelgemisch handeln kann, können in dem Primer Polymere, insbesondere als Klebstoff oder als Haftklebstoff geeignete oder darin enthaltene Polymere, gelöst sein. Der Feststoffgehalt einer solchen Lösung (Polymergehalt) kann vorzugsweise von 10 bis 50 Gew.-%, weiter vorzugsweise von 15 - 25 Gew.-% betragen. Besonders geeignet sind Polymere der folgenden Gruppe oder Copolymere mit Elementen der folgenden Gruppe einzeln oder im Gemisch. Methacrylat, Acrylnitril, Vinylchrorid und Vinylacetat.

Unabhängig vom Material der Verstärkung kann als fakultative Maßnahme die erste Polyurethanlage mit einem Primer bestrichen bzw. behandelt werden, wie oben näher beschrieben, und zwar wenigstens an den Positionen, an denen die beiden Polyurethan-Lagen um die zwischen diesen Lagen liegende Verstärkung herum in Kontakt treten, oder alternativ ganzflächig. Vorzugsweise wird dieser Primer zur besseren Verbindung beider PU-Lagen außerhalb der Fläche, auf der die Verstärkung aufliegt, aufgetragen - dies kann zum Beispiel unmittelbar nach dem Aufsetzen der Verstärkung erfolgen - und wenigstens im Randbereich der ersten PU-Lage, d.h. am Schaft- oder Orthesenrand.

Bei einem derartigen, zwischen zwei PU-Lagen wirksamen Primer kann es sich bevorzugt um eine Lösung einer reaktiven PU-Komponente handeln, besonders bevorzugt um eine isocyanathaltige Lösung. Besonders bevorzugt sind Stoffe der folgenden Gruppe, einzeln oder im Gemisch: MDI, 1,1-Methylenbisisocyanatbenzol und 2,2,2,4-Methylen-diphenyldiisocyanat. Als Lösungsmittel sind besonders einzelne Lösungsmittel oder Gemische aus der Gruppe der Kohlenwasserstoffe geeignet, einschließlich aromatischer Kohlenwasserstoffe-Besonders-bevorzugt ist derzeit ein Einzellösungsmittel oder ein Lösungsmittelgemisch aus wenigstens zwei Bestandteilen der folgenden Gruppe: Xylol, Naphthapetroleum, Trimethylbenzol, Ethylbenzol;

Die Erfindung umfasst zur Lösung der Aufgabe auch allgemein eine mit dem Verfahren herstellbare Halterung, die am menschlichen Körper anzubringen ist und die in ihrem Kernbereich eine gitter-oder rahmenartige Verstärkung aus faserverstärktem Kunststoff mit einer zu deren unmittelbarer Umgebung relativ höheren Steifigkeit enthält, wobei die Verstärkung von einem einheitlich zu Ende reagierten Bereich aus einem Gießharz mit relativ zur Verstärkung geringerer Steifigkeit umgeben und in ihren Ausnehmungen durchdrungen ist. Vorzugsweise ist die gitter- oder rahmenförmige Verstärkung eine nach dem Aushärten gefensterte über einer ersten Polyurethanlage geformte oder gegossene verstärkte Polyacrylatlage.

Das Gießharz ist vorzugsweise, wie oben ausgeführt, ein polyurethyanbasiertes Material, vorzugsweise ein solches auf Basis aliphatischer oder vorwiegend aliphatischer Isocyanate. Das PU-basierte Material kann insbesondere ein Laminat aus dem PU und einem textilen, vorzugsweise gewirkten Material sein.
Die Halterung ist bevorzugt ein Prothesenschaft oder ein Orthesenteil.

Im Folgenden wird die Erfindung anhand von Beispielen und der Zeichnung näher beschrieben. Die Beispiele und Figuren zum Schaftguss dienen allein der Illustration und nicht der Beschränkung der oben dargelegten Erfindung.

In der Zeichnung zeigen:
- Fig. 1 a-e): den Aufbau eines Prothesenschaftes nach dem Verfahren, wobei der Schaft in verschiedenen Stadien im Querschnitt dargestellt ist;
- Fig. 2a u. b): Seitenansichten zweier Schaftbeispiele

### BEISPIEL 1

### Materialien:

1.PU
   HDI-basiertes Mehrkomponentensystem
2. Platzhaltermaterial
   elastische, gewirkte Strümpfe aus Polyamid
3. Verstärkung
   carbonverstärktes Polyacrylat (z.B. Bayer oder Röhm Chemie)
4. Trennfolien aus PVA

### Verfahren

Es wird in üblicher und bekannter Weise ein Positivabguss eines Stumpfes hergestellt (Für die leichtere Handhabung kann eine Stange als Griffstange eingearbeitet werden) Auf den Positivabguss wird eine Trennfolie aufgelegt. Auf diese Trennfolie wird das erste Platzhaltermaterial für die erste PU-Lage aufgebracht. Hierfür werden Strümpfe in mehreren Schichten aufgezogen und geformt. Im Gegensatz zu bekannten Gießverfahren, wird das Platzhaltermaterial nun nicht mit Gießharz getränkt, sondern stattdessen mit einer weiteren Folie überzogen. Hierauf wird in üblicher Weise der Verstärkungsrahmen gearbeitet: Der Verstärkungsbereich wird mit Carbonmatten abgedeckt und mit Acrylharz getränkt. Die Verstärkung wird aushärten gelassen und danach abgenommen. Die dann oben liegende Trennfolie wird abgezogen. Die Verstärkung wird wie gewünscht bearbeitet und gefenstert (ggf. auch mit Primer bestrichen). Nun erst wird die unter der Verstärkung liegende PU-Schicht gegossen. Nachdem sich dieses Polyurethan soweit verfestigt hat, dass es eine hinreichende Formstabilität besitzt und noch nicht ausgehärtet ist, wird (ggf. mit Primer bestrichen und) zügig die gefensterte Verstärkung aufgesetzt, die Platzhalterschicht für die sich anschließende PU-Lage aufgebracht (wie oben schon für die erste Schicht beschrieben, ggf. wird mit Primer behandelt) und die außen um die Verstärkung sich erstreckende PU-Schicht gegossen. Beide PU-Schichten können nun gemeinsam aushärten und sich verbinden. Zusätzliche Schichten sind möglich. Elemente wie Ventile, Stutzen, Pendelaufhängung usw. werden wie üblich angebracht.

### BEISPIEL 2

Wie Beispiel 1, zusätzlich mit Primer für eine Polyacrylat-Verstärkung

Das Acrylharz der Verstärkung wird aus einer Mischung aus Methylmethacrylat und vorpolymerisiertem PMMA mit Benzoylperoxid (50 %ig) radikalisch gehärtet. Die gefensterte Verstärkung liegt im vollständig durchgehärtetem Zustand vor.

Die Verstärkung wird auf ihrer gesamten Oberfläche mit einem Primer behandelt, nämlich hier mit einer polyvinylchlorid- und/oder polyvinylacetathaltigen Lösung mit 2-Butanon als Lösungsmittel. Bevorzugt enthält die Lösung ein epoxidiertes Vinylchlorid-Vinylacetat-Copolymer.

Es ist vorteilhaft, den Primer zunächst physikalisch antrocknen oder das Lösungsmittel erst etwas abdunsten zu lassen, bevor die Verstärkung auf die unterliegende PU-Lage aufgesetzt wird.

### BEISPIEL 3

Wie Beispiel 1 oder Beispiel 2, zusätzlich mit einem Primer zwischen den beiden PU-Lagen

Die erste PU-Lage, die durch Tränken des Platzhaltermaterials nach Abnehmen der Verstärkung erstellt wurde, wird unmittelbar vor erneutem Aufsetzen der inzwischen gefensterten Verstärkung mit einem Primer bestrichen, der aus einer der folgenden Lösungen besteht, die auch in verschiedenen Mischungen untereinander angewendet werden können:
1) MDI (Diphenylmethan-4,4'-diisocyanat) in einer Mischung aus Xylol, Naphthapetroleum, Trimethylbenzol, Ethylbenzol zu gleichen Teilen;
2) 1,1-Methylenbisisocyanatobenzol in einer Mischung aus Xylol, Naphthapetroleum, Trimethylbenzol, Ethylbenzol zu gleichen Teilen;
3) 2,2,2,4-Methylen-dihenyldiisocyanat in einer Mischung aus Xylol, Naphthapetroleum, Trimethylbenzol, Ethylbenzol zu gleichen Teilen.

Anschließend wird die gefensterte Verstärkung aufgesetzt und die auf der Verstärkung aufliegende PU-Lage erstellt.

### BEISPIEL 4

Variante zu Beispiel2: Nach Aufsetzen der wie in Beispiel 2 mit Primer versehenen Verstärkung wird die frei gebliebene Fläche der ersten PU-Lage mit einem Primer wie in Beispiel 3 bestrichen. Die weitere Verarbeitung erfolgt wie in Beispiel 1.

Nachfolgend wird das Verfahren nochmals beispielhaft für einen Prothesenschaft mit Hilfe der Figuren erläutert.

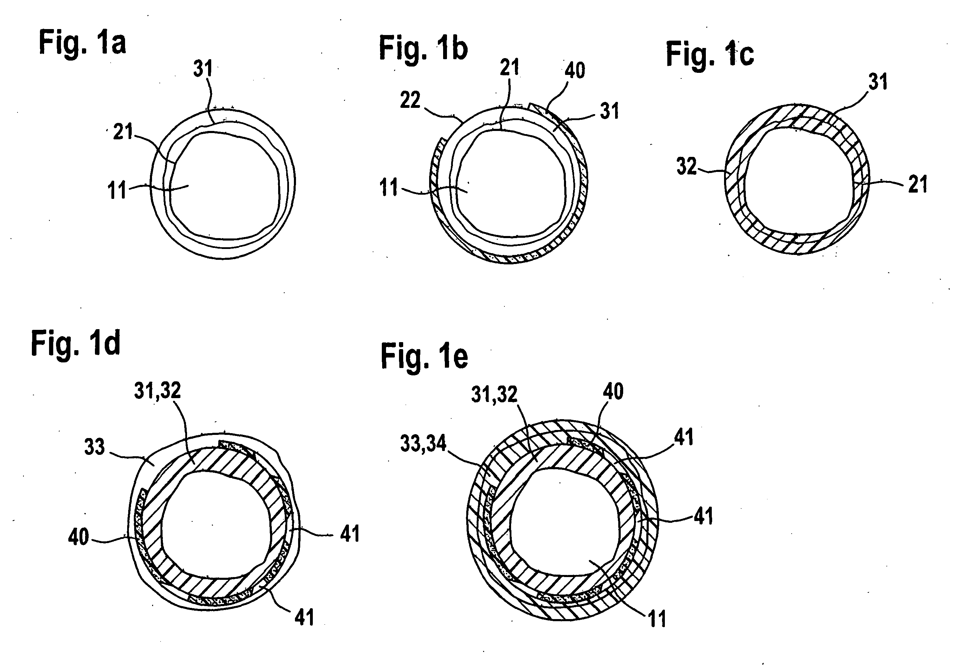

Figur 1 zeigt den Aufbau eines Schaftes in verschiedenen Stadien jeweils im Querschnitt.

Aus Figur 1a ist zu erkennen, wie der Positivabguss eines Stumpfes 11 von der ersten Lage aus Platzhaltermaterial umgeben ist. Auf dem Positivabguss 11 liegt eine erste Trennfolie 21 auf, hierauf folgen mehrere Schichten elastischer gewirkter Strümpfe als erstes Platzhaltermaterial 31.

In Figur 1b wurde hierüber eine zweite Trennfolie 22 gezogen und auf dieser eine Verstärkung 40 aus Carbonmatten und Acrylharz erstellt. Die ausgehärtete Verstärkung 40, die in diesem Beispiel schalenförmig um den Schaft reicht, wird abgenommen und in üblicher Weise bearbeitet (gefenstert), so dass ein Verstärkungsrahmen entsteht.

Figur 1c zeigt die gegossene, der Verstärkung 40 später unterliegende, gliedmaßenseitige PU-Lage 32, die das durchtränkte erste Platzhaltermaterial 31 enthält. Die zweite Trennfolie 22 wurde entfernt. Nachdem sich das PU soweit verfestigt hat, dass es eine hinreichende Formstabilität besitzt und noch nicht ausgehärtet ist, wird die gefensterte Verstärkung 40 aufgesetzt, die mit einem Primer bestrichen sein kann.

Figur 1d zeigt die Verstärkung 40 und die Fensterbereiche 41. Nun wird die Platzhalterschicht 33 für die weitere PU-Lage aufgebracht, indem wiederum elastische gewirkt Strümpfe über die erste PU-Lage und die gitterförmige Verstärkung 40 aufgezogen werden.

Figur 1e schließlich zeigt zusätzlich die sich außen um die Verstärkung erstreckende, gegossene PU-Schicht 34, die das Platzmaterial 33 enthält. Die Schichten 34 und 32 berühren sich um die Verstärkung 40 herum und in den Fensterbereichen 41. Beide PU-Schichten können nun gemeinsam aushärten und sich verbinden. Gegebenenfalls vorhandene zusätzliche Schichten, wie Abdeckungen, sind nicht dargestellt, ebenso wie Ventile, Stutzen etc, die in bekannter Weise angebracht werden können.

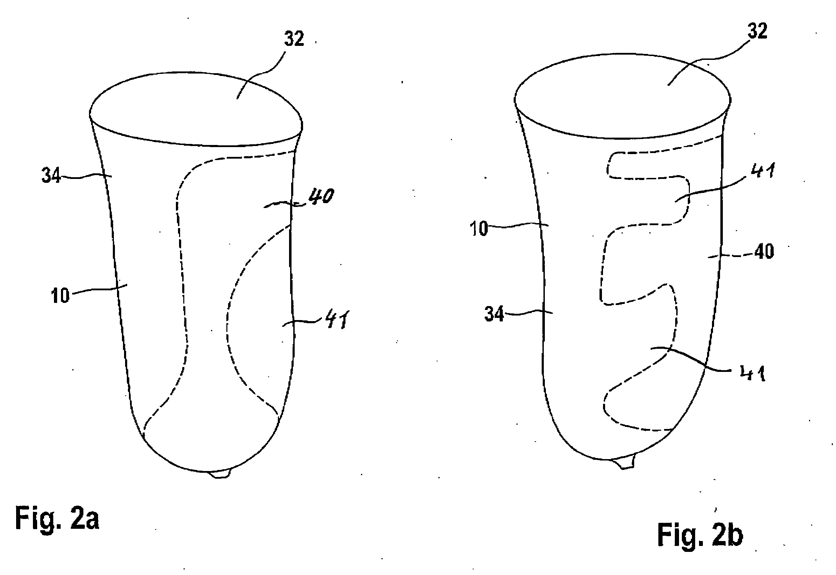

Figuren 2a und 2b zeigen zwei Beispiele für Protheseschäfte 10 in Seitenansicht, die sich lediglich in der Form der Verstärkung 40 unterscheiden. Wie zu sehen, können hier verschiedenste Formen gewählt und mit dem Verfahren realisiert werden. Die PU-Schichten 32 und 34 verbinden sich überall dort, wo keine Verstärkung zwischen ihnen zu liegen kommt.

## Patentansprüche

1. Verfahren zum Gießen einer am menschlichen Körper anzubringenden Halterung in Form eines Prothesenschaftes (10) oder eines Orthesenteils mit in Ihrem Kernbereich liegender, gitter- oder rahmenförmiger Verstärkung (40) und dem grundsätzlichen Schichtaufbau: unterliegende Polyurethan(PU)-Lage (32), Verstärkung im Kernbereich, aufliegende Polyurethan(PU)-Lage (34),
**gekennzeichnet durch** folgende Schritte in der angegebenen Reihenfolge:
a) an der Position einer der Verstärkung unterliegenden PU-Lage (32) wird ein mit dem noch nicht ausreagierten Polyurethan tränkbares Platzhaltermaterial (31) in Form der gewünschten gliedmaßenseitigen PU-Lage (32) aufgebracht,
b) darauf wird die gitter- oder rahmenförmige Verstärkung (40) erstellt,
c) die Verstärkung (40) wird abgenommen,
d) die erste Polyurethanlage (32) wird unter Durchtränken des Platzhaltermaterials (31) gegossen,
e) die Verstärkung (40) wird auf die Polyurethanoberfläche der ersten Polyurethanlage (32) in einem Polymerisationszustand nach Erreichen der Formstabilität wieder aufgesetzt,
f) ein weiteres mit dem noch nicht ausreagierten Polyurethan tränkbares Platzhaltermaterial (33) in Form der gewünschten weiteren PU-Lage (34) wird aufgebracht und
g) die weitere PU-Lage (34) wird unter Durchtränken des zweiten Platzhaltermaterials (33) gegossen, so dass sich die beiden PU-Lagen (32; 34) dort wo sie in Kontakt treten beim Aushärten des Polyurethans der aufliegenden PU-Lage (34) mit Hilfe eines zwischen den PU-Lagen aufgebrachten Primers und/oder **durch** gemeinsames Aushärten beider PU-Lagen reaktiv verbinden und die Verstärkung (40) einschließen.

2. Verfahren nach Anspruch 1, **dadurch gekennzeichnet, dass** genau die folgenden Schritte durchgeführt werden:
a) an der Position einer der Verstärkung (40) unterliegenden PU-Lage (32) wird ein mit dem noch nicht ausreagierten Polyurethan tränkbares Platzhaltermaterial (31) in Form der gewünschten gliedmaßenseitigen PU-Lage (32) aufgebracht,
b) darauf wird die gitter- oder rahmenförmige Verstärkung (40) erstellt,
c) die Verstärkung (40) wird abgenommen,
d) die erste Polyurethanlage (32) wird unter Durchtränken des Platzhaltermaterials (31) gegossen,
e) die Verstärkung (40) wird auf die Polyurethanoberfläche der ersten Polyurethanlage (32) in einem Polymorisationszustand nach Erreichen der Formstabilität wieder aufgesetzt und es wird wenigstens auf dem Randbereich der ersten PU-Lage (32), außerhalb der Fläche, auf der die Verstärkung (40) aufliegt, ein Primer aufgetragen,
f) ein weiteres mit dem noch nicht ausreagierten Polyurethan tränkbares Platzhaltermaterial (33) in Form der gewünschten weiteren PU-Lage (34) wird aufgebracht und
g) die weitere PU-Lage (34) wird unter Durchtränken des zweiten Platzhaltermaterials (33) gegossen, so dass sich die beiden PU-Lagen (32; 34)dort wo sie in Kontakt treten beim Aushärten des Polyurethans der aufliegenden PU-Lage (34) mit Hilfe des zwischen den PU-Lagen aufgebrachten Primers reaktiv verbinden und die Verstärkung (40) einschließen.

3. Verfahren nach Anspruch 1 oder 2, **dadurch gekennzeichnet, dass** das Polyurethan für die die Verstärkung (40) umgebenden Lagen (32; 34) ein solches auf Basis aliphatischer Isocyanate ist, vorzugsweise auf Basis von HDI.

4. Verfahren nach einem der Ansprüche 1 bis 3, **dadurch gekennzeichnet, dass** die gitter- oder rahmenförmige Verstärkung (40) eine nach dem Aushärten gefensterte über dem Platzhaltermaterial (31) der ersten PU-Lage (32) geformte oder gegossene verstärkte Polyacrylatlage ist.

5. Verfahren nach Anspruch 4, **dadurch gekennzeichnet, dass** die gefensterte Polyacrylatlage carbonfaserverstärkt ist.

6. Verfahren nach einem der Ansprüche 1 bis 5, **dadurch gekennzeichnet, dass** für den Gießvorgang um das Platzhaltermaterial (31; 33) eine Trennfolie (21; 22) gelegt wird, die nach dem Aushärten des Gießharzes oder nach Erreichen der Formstabilität der Lage abgezogen wird.

7. Verfahren nach einem der Ansprüche 1 bis 6, **dadurch gekennzeichnet, dass** das Platzhaltermaterial (31; 33) ein in wenigstens einer Schicht aufgebrachtes textiles, vorzugsweise gewirktes Material ist.

8. Verfahren nach Anspruch 7, **dadurch gekennzeichnet, dass** das textile Material strumpf- oder sockenförmig ist und vorzugsweise mehrere Strümpfe oder Socken übereinander gezogen werden.

9. Verfahren nach einem der Ansprüche 1 bis 8, **dadurch gekennzeichnet, dass** das Platzhaltermaterial (31; 33) aus einer porösen Manschette besteht, vorzugsweise aus einem Polyurethanschaum.

10. Verfahren nach einem der Ansprüche 1 bis 9, **dadurch gekennzeichnet, dass** die erste PU-Lage (32) auf einen Positivabguss (11) des mit der Halterung zu versehenden Stumpfes oder Gliedmaßenteils aufgebaut wird, vorzugsweise über einer Trennfolie (21) oder einem Trennmittel.

11. Verfahren nach einem der Ansprüche 1 bis 10, **dadurch gekennzeichnet, dass** in einem zusätzlichen Zwischenschritt auf das Platzhaltermaterial (31; 33) eine Negativform aufgebaut wird, das Platzhaltermaterial entfernt wird und die PU-Lage (32; 34) in den Hohlraum zwischen Negativform und Unterlage des entfernten Platzhaltermaterials gegossen wird.

12. Verfahren nach einem der Ansprüche 1 bis 11, **dadurch gekennzeichnet dass** zusätzlich auf die gegitterte oder rahmenförmige Verstärkung (40) ein Primer, Haftvermittler oder Klebstoff, vorzugsweise aus einer Polymerlösung, aufgebracht wird.

## Claims

1. Method for casting a mounting which is to be attached to the human body and is in the form of a prosthesis shaft (10) or an orthosis part with, located in its core region, a grid-shaped or frame-shaped reinforcement (40) and with the basic layer structure: lower polyurethane (PU) layer (32), reinforcement In the core region, superposed polyurethane (PU) layer (34), **characterised by** the following steps in the given order:
a) a spacer material (31), which can be saturated with the incompletely reacted polyurethane and is in the form of the desired limb-proximal PU layer (32), is applied at the position of a PU layer (32) lying below the reinforcement,
b) the grid-shaped or frame-shaped reinforcement (40) is produced thereon,
c) the reinforcement (40) is removed,
d) the first polyurethane layer (32) is cast with saturation of the spacer material (31),
e) the reinforcement (40) is again placed on the polyurethane surface of the first polyurethane layer (32) in a polymerisation state after reaching dimensional stability,
f) a further spacer material (33), which can be saturated with the incompletely reacted polyurethane and is in the form of the desired further PU layer (34), is applied and
g) the further PU layer (34) is cast with saturation of the second spacer material (33), so the two PU layers (32; 34), where they come into contact during the hardening of the polyurethane of the superposed PU layer (34), bond reactively with the aid of a primer applied between the PU layers and/or by hardening of the two PU layers together, and enclose the reinforcement (40).

2. Method according to claim 1, **characterised in that** precisely the following steps are carried out:
a) a spacer material (31), which can be saturated with the incompletely reacted polyurethane and is in the form of the desired limb-proximal PU layer (32), is applied at the position of a PU layer (32) lying below the reinforcement (40),
b) the grid-shaped or frame-shaped reinforcement (40) is produced thereon,
c) the reinforcement (40) is removed,
d) the first polyurethane layer (32) is cast with saturation of the spacer material (31),
e) the reinforcement (40) is again placed on the polyurethane surface of the first polyurethane layer (32) in a polymerisation state after reaching dimensional stability and a primer is applied at least to the edge region of the first PU layer (32) outside the area, on which the reinforcement (40) rests,
f) a further spacer material (33), which can be saturated with the incompletely reacted polyurethane and is in the form of the desired further PU layer (34), is applied
g) the further PU layer (34) is cast with saturation of the second spacer material (33), so the two PU layers (32; 34), where they come into contact during the hardening of the polyurethane of the superposed PU layer (34), bond reactively with the aid of the primer applied between the PU layers and enclose the reinforcement (40).

3. Method according to claim 1 or 2, **characterised in that** the polyurethane for the layers (32; 34) surrounding the reinforcement (40) is a polyurethane based on aliphatic isocyanates, preferably based on HDI.

4. Method according to any one of claims 1 to 3, **characterised in that** the grid-shaped or frame-shaped reinforcement (40) is a reinforced polyacrylate layer shaped or cast above the spacer material (31) of the first PU layer (32) and windowed after hardening,

5. Method according to claim 4, **characterised in that** the windowed polyacrylate layer is carbon-fibre reinforced.

6. Method according to any one of claims 1 to 5, **characterised in that** a separator foil (21; 22) is placed around the spacer material (31; 33) for the casting process and Is removed after the hardening of the casting resin or after reaching dimensional stability.

7. Method according to any one of claims 1 to 6, **characterised in that** the spacer material (31; 33) is a textile, preferably knitted, material applied in at least one layer.

8. Method according to claim 7, **characterised in that** the textile material is stocking-shaped or sock-shaped and a plurality of stockings or socks are preferably pulled over one another.

9. Method according to any one of claims 1 to 8, **characterised in that** the spacer material (31; 33) consists of a porous sleeve, preferably made of a polyurethane foam.

10. Method according to any one of claims 1 to 9, **characterised in that** the first PU layer (32) is constructed on a positive cast replica (11) of the stump or limb part to be provided with the mounting, preferably over a separator foil (21) or a separating agent.

11. Method according to any one of claims 1 to 10, **characterised in that** a female mould is constructed on the spacer material (31; 33) in an additional intermediate step, the spacer material is removed and the PU layer (32; 34) Is cast into the cavity between the female mould and the substrate of the removed spacer material,

12. Method according to any one of claims 1 to 11, **characterised in that** a primer, adhesion promoter or adhesive, preferably made of a polymer solution, is additionally applied to the grid-like or frame-shaped reinforcement (40).

## Revendications

1. Procédé de moulage d'une fixation à mettre en place sur le corps humain, en forme d'emboîture (10) ou de partie d'orthèse, comprenant un renforcement (40) en forme de grille ou de cadre s'étendant dans sa région de coeur, et présentant la structure de base en couches : couche de polyuréthane (PU) (32) en-dessous, renforcement dans la région de coeur, couche de polyuréthane (PU) (34) au-dessus, **caractérisé par** les étapes suivantes dans l'ordre indiqué :
a) à l'emplacement d'une couche de PU (32) située en-dessous du renforcement, on pose un matériau garde-place (31) imprégnable par le polyuréthane n'ayant pas encore fini de réagir, de la forme de la couche PU (32) côté membre souhaitée,
b) on réalise par-dessus le renforcement (40) en forme de grille ou de cadre,
c) on enlève le renforcement (40),
d) on coule la première couche de polyuréthane (32) par imprégnation du matériau garde-place (31),
e) on replace le renforcement (40) sur la surface de polyuréthane de la première couche de polyuréthane (32) dans un état de polymérisation après avoir atteint la stabilité de forme,
f) on pose un autre matériau garde-place (33) impégnable par le polyuréthane n'ayant pas encore fini de réagir, de la forme de la couche PU (34) suivante souhaitée, et
g) on coule la couche de PU (34) suivante par imprégnation du deuxième matériau garde-place (33), de sorte que les deux couches de PU (32 ; 34) se lient de façon réactionnelle et enferment le renforcement (40), là où elles entrent en contact lors du durcissement du polyuréthane de la couche de PU (34) du dessus, à l'aide d'un apprêt posé entre les couches de PU et/ou par durcissement commun des deux couches de PU.

2. Procédé selon la revendication 1, **caractérisé en ce qu'**on met en oeuvre précisément les étapes suivantes :
a) à l'emplacement d'une couche de PU (32) située en-dessous du renforcement (40), on pose un matériau garde-place (31) imprégnable par le polyuréthane n'ayant pas encore fini de réagir, de la forme de la couche PU (32) côté membre souhaitée,
b) on réalise par-dessus le renforcement (40) en forme de grille ou de cadre,
c) on enlève le renforcement (40),
d) on coule la première couche de polyuréthane (32) par imprégnation du matériau garde-place (31),
e) on replace le renforcement (40) sur la surface de polyuréthane de la première couche de polyuréthane (32) dans un état de polymérisation après avoir atteint la stabilité de forme, et on pose un apprêt au moins sur la région du bord de la première couche de PU (32), en-dehors de la surface sur laquelle se trouve le renforcement (40),
f) on pose un autre matériau garde-place (33) imprégnable par le polyuréthane n'ayant pas encore fini de réagir, de la forme de la couche PU (34) suivante souhaitée, et
g) on coule la couche de PU (34) suivante par imprégnation du deuxième matériau garde-place (33), de sorte que les deux couches de PU (32 ; 34) se lient de façon réactionnelle et enferment le renforcement (40), là où elles entrent en contact lors du durcissement du polyuréthane de la couche de PU (34) du dessus, à l'aide de l'apprêt posé entre les couches de PU.

3. Procédé selon la revendication 1 ou 2, **caractérisé en ce que** le polyuréthane pour les couches (32 ; 34) entourant le renforcement (40) est du type à base d'isocyanates aliphatiques, de préférence à base de HDI.

4. Procédé selon l'une quelconque des revendications 1 à 3, **caractérisé en ce que** le renforcement (40) en forme de grille ou de cadre est une couche de polyacrylate renforcée moulée ou coulée sur le matériau garde-place (31) de la première couche de PU (32), fenêtrée après le durcissement.

5. Procédé selon la revendication 4, **caractérisé en ce que** la couche de polyacrylate fenêtrée est renforcée à la fibre de carbone.

6. Procédé selon l'une quelconque des revendications 1 à 5, **caractérisé en ce que** pour le processus de coulée autour du matériau garde-place (31 ; 33), on dépose un film de séparation (21 ; 22) que l'on enlève après le durcissement de la résine coulée ou après avoir atteint la stabilité de forme de la couche.

7. Procédé selon l'une quelconque des revendications 1 à 6, **caractérisé en ce que** le matériau garde-place (31 ; 33) est un matériau textile posé en au moins une couche, de préférence maillé.

8. Procédé selon la revendication 7, **caractérisé en ce que** le matériau textile est en forme de bas ou de chaussette et de préférence on enfile plusieurs bas ou chaussettes les uns sur les autres.

9. Procédé selon l'une quelconque des revendications 1 à 8, **caractérisé en ce que** le matériau garde-place (31 ; 33) consiste en une manchette poreuse, de préférence formée d'une mousse de polyuréthane.

10. Procédé selon l'une quelconque des revendications 1 à 9, **caractérisé en ce que** l'on construit la première couche de PU (32) sur un moulage positif (11) du moignon ou de la partie de membre à munir de la fixation, de préférence sur un film de séparation (21) ou sur un moyen de séparation.

11. Procédé selon l'une quelconque des revendications 1 à 10, **caractérisé en ce que** dans une étape intermédiaire supplémentaire, on construit un moule négatif sur le matériau garde-place (31 ; 33), on enlève le matériau garde-place et on coule la couche de PU (32 ; 34) dans l'espace creux entre le moulage négatif et le support du matériau garde-place enlevé.

12. Procédé selon l'une quelconque des revendications 1 à 11, **caractérisé en ce que** qu'on pose en outre sur le renforcement (40) en forme de grille ou de cadre un apprêt, un agent adhésif, ou une colle, de préférence formé par une solution de polymère.
